(19)

Europäisches
Patentamt

European
Patent Office

Office européen
des brevets

(11) **EP 4 359 112 B1**

(12) **EUROPEAN PATENT SPECIFICATION**

(45) Date of publication and mention
of the grant of the patent:
**23.04.2025  Bulletin 2025/17**

(21) Application number: **22733486.9**

(22) Date of filing: **20.06.2022**

(51) International Patent Classification (IPC):
**B01D 53/52** *(2006.01)*     **B01D 53/84** *(2006.01)*
**B01D 53/96** *(2006.01)*     **C02F 3/34** *(2023.01)*
**C01B 17/05** *(2006.01)*     **C12N 1/20** *(2006.01)*
**C12P 1/04** *(2006.01)*     **B01J 8/00** *(2006.01)*
**B01J 19/24** *(2006.01)*     **C12M 1/12** *(2006.01)*

(52) Cooperative Patent Classification (CPC):
**B01D 53/52; B01D 53/84; B01D 53/96;**
**C01B 17/05; C02F 3/345; C12N 1/20; C12P 3/00;**
B01D 53/526; B01D 2251/11; B01D 2251/304;
B01D 2251/306; B01D 2251/606; B01D 2251/95;
C02F 2101/101; C02F 2103/18;          (Cont.)

(86) International application number:
**PCT/NL2022/050345**

(87) International publication number:
**WO 2022/271011 (29.12.2022 Gazette 2022/52)**

(54) **A PROCESS TO CONTINUOUSLY TREAT A HYDROGEN SULPHIDE COMPRISING GAS AND SULPHUR RECLAIMING FACILITIES**

VERFAHREN ZUR KONTINUIERLICHEN BEHANDLUNG VON SCHWEFELWASSERSTOFF ENTHALTENDEM GAS UND SCHWEFELRÜCKGEWINNUNGSANLAGEN

PROCESSUS POUR TRAITER EN CONTINU UN GAZ COMPRENANT DU SULFURE D'HYDROGÈNE ET DES INSTALLATIONS DE RÉCUPÉRATION DE SOUFRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **21.06.2021  NL 2028503**

(43) Date of publication of application:
**01.05.2024  Bulletin 2024/18**

(73) Proprietor: **Paques I.P. B.V.**
**8561 EL Balk (NL)**

(72) Inventors:
• **KLOK, Johannes Bernardus Maria**
**8561 EL Balk (NL)**
• **MEHTA, Dhaval**
**8561 EL Balk (NL)**
• **DIJKMAN, Hendrik**
**8561 EL Balk (NL)**
• **MOL, Annemerel Rozemarijn**
**8561 EL Balk (NL)**
• **VAN DER WEIJDEN, Renata Dorothea**
**8561 EL Balk (NL)**
• **BUISMAN, Cees Jan Nico**
**8561 EL Balk (NL)**

(74) Representative: **Nederlandsch Octrooibureau**
**P.O. Box 29720**
**2502 LS The Hague (NL)**

(56) References cited:
**EP-A1- 3 034 157        WO-A1-2004/091754**
**WO-A1-2015/114069    WO-A1-92/10270**
**JP-A- 2012 193 252**

EP 4 359 112 B1

**(Cont. next page)**

(52) Cooperative Patent Classification (CPC): (Cont.)
Y02A 50/20

**Description**

The invention is directed to a process to continuously treat a hydrogen sulphide comprising gas, said process comprising the following steps: (a) contacting the hydrogen sulphide comprising gas with an aqueous alkaline liquid further comprising sulphide-oxidising bacteria and elemental sulphur particles, thereby producing a loaded aqueous liquid, (b) contacting the loaded aqueous liquid with an oxidant wherein sulphide is oxidised to elemental sulphur by the sulphide-oxidising bacteria, thereby producing an enriched aqueous liquid comprising an increased amount of elemental sulphur particles and (c) separating elemental sulphur particles from the enriched aqueous liquid. The invention is also directed to sulphur reclaiming process facilities.

Such a biological desulfurization process is described in WO92/10270. The process as described in this publication has been applied in already more than 250 commercial installations worldwide. Sulphur particle separation, however, remains a challenge; a fraction of the sulphur particles is often too small for liquid-solid separation with conventional separation technology.

Sulfur is the 10th most abundant element in the universe and plays a vital role in the Earth's ecosystem through the (bio)chemical sulfur cycle. It can be present in various oxidation states, from hydrogen sulphide ($H_2S$) being the most reduced state (-2) to sulfate ($SO_4^{2-}$) the most oxidized state (+6). Elemental sulfur (S with an oxidation state of zero), can be recovered from hydrogen sulphide comprising gas using the process as described in WO92/10270. Main advantages of this process in comparison to chemical and physical alternatives are operation at ambient pressure and temperature and without toxic chemicals. In the process of WO92/10270 $H_2S$ as is absorbed in a moderately alkaline solution where it reacts to soluble bisulphide and is subsequently oxidized by a mixed culture of sulphide-oxidizing bacteria to elemental sulfur. The elemental sulphur is predominantly present in the form of orthorhombic $\alpha$-$S_8$. Next to formation of elemental sulfur, oxidized by-products such as sulfate and thiosulfate are formed due to overexposure to dissolved oxygen wherein sulfate is formed biologically and thiosulfate is formed abiotically. These compounds are undesirable as they lead to acidification and consequently addition of chemicals is needed to neutralize the process solution.

Although the above described biological desulfurization process has been intensively studied the sulphur settleability is still a major challenge. Sulphur is found to settle poorly and the settleability of the produced sulphur in commercial processes fluctuates over time. Poorly settleable sulphur may lead to hampered process operation, as it accumulates in the system. Accumulated sulphur can cause problems such as clogging of pumps and pipes, and under high concentrations to foaming as well. Moreover, small sulphur particles are more prone to side reactions such as oxidation, due to their larger relative surface area.

The object of this invention is to provide a process and sulphur reclaiming facility which does not have the afore mentioned problems regarding poor and fluctuating sulphur settleability.

This is provided with the following process. A process to continuously treat a hydrogen sulphide comprising gas, said process comprising the following steps:

(a) contacting the hydrogen sulphide comprising gas with an aqueous alkaline liquid further comprising sulphide-oxidising bacteria and elemental sulphur particles, thereby producing a loaded aqueous liquid comprising dissolved sulphide, polysulphide compounds, sulphide-oxidising bacteria and elemental sulphur particles and a gas having a lower content of hydrogen sulphide, and passing the loaded aqueous liquid through a polysulphide reactor zone comprising one or more plug flow reactor zones,
(b) contacting the loaded aqueous liquid with an oxidant to enable the sulphide-oxidising bacteria to oxidise sulphide to elemental sulphur, thereby producing an enriched aqueous liquid comprising an increased amount of elemental sulphur particles, and
(c) separating elemental sulphur particles from the enriched aqueous liquid, wherein the residence time of the loaded aqueous liquid between its preparation in step (a) and its supply to step (b) is between 3 and 45 minutes, and wherein the content of the elemental sulphur as part of the polysulphide compounds in the loaded aqueous liquid [$S^0$ in $S_x^{2-}$] as supplied to step (b) is above 0.7 mM.

This is also provided by the sulphur reclaiming process facilities according to claims 10 and 11.

Applicants have found that the settleability of elemental sulphur is significantly improved when the process is performed as claimed. Results show that agglomeration of sulphur particles is promoted while the presence of very small single particles is lower compared to the prior art process conditions. It is surprising that a high content of polysulphide compounds in the loaded aqueous liquid is beneficial because polysulphide formation in the bioreactor of step (b) was deemed unwanted, as it indicates a poor operating condition. Furthermore, polysulphides are known to be more sensitive for over oxidation than sulphide, which results in a lower efficiency of elemental sulphur formation/ regeneration of caustic which is unwanted. Applicant believes that the improvement regarding settlement of elemental sulphur results from the fact that very small sulphur particles are removed in step (a) and before performing step (b) as a result of the presence of polysulphides. Due to polysulphide formation the remaining smallest particles in the liquid are believed to have a higher tendency to agglomerate resulting in an improved settlement of elemental sulphur.

Without wishing to be bound by the following theory applicant believes that the improved settlement of elemental sulphur can be explained by the following equilibrium between polysulphide and S8 rings:

$$HS^- + (x-1)/8\ S_8 <--> S_x^{2-} + H^+ \qquad (1)$$

When the content of polysulphide, $[S_x^{2-}]$, is high the formation of dissolved S8 rings will also be promoted resulting in a so-called super saturation of dissolved S8 rings which in turn results in the desired formation of aggregates under so-called polysulphidic conditions. These polysulphidic conditions are expressed by the following formula (2) wherein $[S^0\ in\ S_x^{2-}]$ is the content of elemental sulphur as part of the polysulphide compounds $[S_x^{2-}]$ in the loaded aqueous liquid as supplied to step (b):

$$\left[S^0\ in\ S_x^{2-}\right] \geq 1.7 * [HS^-] * 10^{(-9.17+pH)} \qquad (2)$$

wherein $[S^0\ in\ S_x^{2-}]$ is the content of elemental sulphur as part of the polysulphide compounds in the loaded aqueous liquid as supplied to step (b) expressed in mM S, $[HS^-]$ is the sulphide concentration expressed in mM of the loaded aqueous liquid as supplied to step (b), and pH is the pH of the loaded aqueous liquid as supplied to step (b).

Even more preferably, the polysulphidic conditions are expressed by the following formula (3)

$$[S^0\ in\ S_x^{2-}] \geq 2.8 * [HS^-] * 10^{(-9.17+pH)} \qquad (3)$$

Typically,

$$[S^0\ in\ S_x^{2-}] \leq 6.0 * [HS^-] * 10^{(-9.17+pH)} \qquad (4)$$

The content of the elemental sulphur as part of the polysulphide compounds in the loaded aqueous liquid $[S^0\ in\ S_x^{2-}]$ as supplied to step (b) is above 0.7 mM and preferably above 1 mM and even more preferably above 1.5 mM.

The content of the elemental sulphur as part of the polysulphide compounds in the loaded aqueous liquid $[S^0\ in\ S_x^{2-}]$ depends on the content of polysulphide, $[S_x^{2-}]$ and the average chain length x according to the following formula:

$$\left[S^0\ in\ S_x^{2-}\right] = (x-1)* \left[S_x^{2-}\right] \qquad (3)$$

For example, for a polysulphide wherein x=4 according to the following formula S-S-S-S$^{2-}$ and present in a concentration $[S_x^{2-}]$ of 1.5 mM the content of elemental sulphur as part of the polysulphide compounds $[S^0\ in\ S_x^{2-}]$ is:

$$(4-1)\ x1.5\ mM=4.5\ mM.$$

Polysulphides are formed by reaction between bisulphide and elemental sulphur. The polysulphide itself may also react with elemental sulphur. It is believed that due to the high surface to volume ratio of the very small elemental sulphur particles these particles are selectively consumed. This chemical reaction obviously also takes place in the prior art processes. However in the prior art processes the concentration of polysulphides does not reach the level that is achieved in the present process and that results in the improved sulphur settlement. For this reason it is preferred to operate the process according to this invention for a period of at least 1 week such that the daily average content of elemental sulphur as part of the polysulphide compounds in the loaded aqueous liquid $[S^0\ in\ S_x^{2-}]$ as supplied to step (b) is above 0.7 mM, preferably above 1 mM and even more preferably above 1.5 mM. More preferably to operate the process according to this invention for a period of at least 1 week under the polysulphidic conditions as expressed by the above formula (2).

The hydrogen sulphide comprising gas may be any gas comprising such a compound. The hydrogen sulphide comprising gas may also comprise carbon dioxide, nitrogen, hydrogen, small amounts of oxygen, water vapour and gaseous hydrocarbons, such as for example methane, ethane, propane and/or higher boiling hydrocarbons, and mercaptans and/or other sulphur compounds, for example carbonyl sulphide. Such a gas may be natural gas, bio-gas from for example anaerobic wastewater treatment units, refinery off gas, synthesis gas, geothermal gas, landfill gas or acid gas obtained in an amine gas treating process. The invention is especially suited for gasses having a content of carbon dioxide of above 20 vol% and a hydrogen sulphide content of between 0.1 and 3 vol.%. When such gasses are treated by the prior art processes sulphur settleability is especially a challenge. With hindsight it is believed that a low content of sulphur as part of polysulphide at the resulting lower pH and the lower bisulphide content cause a poor settlement of elemental sulphur as also illustrated in Example B. When such gasses are treated by the process of this invention an improved settleability of elemental sulphur is observed.

In step (a) the hydrogen sulphide comprising gas is contacted with an aqueous alkaline liquid further comprising sulphide-oxidising bacteria and at a temperature of preferably between 15 and 48 °C and more preferably between 35 and 45 °C. Such a process is also referred to as an absorption process and is typically performed in an absorption or contacting column where gas and liquid flow counter-currently. Suitably step (a) is performed in a vertical column wherein continuously the hydrogen sulphide comprising gas is fed to the column at a lower position of the column and the aqueous liquid comprising sulphide-oxidising bacteria is continuously fed to a higher position of the column such that a substantially upward flowing gaseous stream contacts a substantially downwards flowing aqueous stream. The column is further provided with an outlet for the loaded aqueous liquid at its lower end and an outlet for treated gas at its upper end.

The aqueous alkaline liquid may be any liquid alkaline absorbent known to be suitable for absorption of hydrogen sulphide. Examples of suitable liquid alkaline absorbents are carbonate, bicarbonate and/or phosphate solutions and more preferably the aqueous liquid is a buffered liquid further comprising sodium carbonate and sodium bicarbonate or potassium carbonate and potassium bicarbonate or their mixtures. The pH of the liquid aqueous alkaline liquid is preferably in the range of from 7 to 10, more preferably of from 7.5 to 9.5. It will be appreciated that in downward direction of the column, the pH of the absorption liquid will decrease due to absorption of hydrogen sulphide and carbon dioxide. The pH of the loaded aqueous liquid produced in step (a) will be typically lower than the pH of the aqueous liquid provided to the absorption column. The pH of the loaded aqueous liquid produced in step (a) may be as low as 6.5 and is preferably in the range of from 6.5 to 9.0.

The pressure in step (a) may be up to 100 bara, preferably of from atmospheric pressure to 80 bara.

The concentration of oxygen and especially dissolved oxygen is low in step (a). The concentration of molecular oxygen in the loaded aqueous liquid produced in step (a) is at most 10 $\mu$M, preferably at most 1 $\mu$M, more preferably at most 0.1 $\mu$M. To achieve these low oxygen contents the oxygen content in the hydrogen sulphide comprising gas is suitably below 3 vol.% and preferably below 1 vol.%.

The sulphide-oxidising bacteria as present in step (a) may be any sulphide-oxidising bacteria, preferably sulphide-oxidising bacteria of the genera *Halothiobacillus, Thioalkalimicrobium, Thioalkalispira, Thioalkalibacter, Thioalkalivibrio, Alkalilimnicola* and related bacteria. The sulphide-oxidising bacteria as present in step (a) may be provided by recirculating the enriched aqueous liquid from step (b) and/or by recirculating aqueous liquid obtained after removal of elemental sulphur particles in step (c).. It has been found that when such sulphide-oxidising bacteria are present in the aqueous alkaline liquid under the above described conditions a very effective absorption of hydrogen sulphide results. The content of sulphide-oxidising bacteria, based on nitrogen content, in the aqueous alkaline liquid in step (a) is preferably greater than 5 mg N/L and lower than 1000 mg N/L and more preferably between 25 and 200 mg N/L.

The loaded aqueous liquid as produced in step (a) comprises dissolved bisulphide, elemental sulphur particles and sulphide oxidising bacteria. The combined concentration of bisulphide, polysulphide compounds, sulphur in sulphide-oxidising bacteria and elemental sulphur in the loaded aqueous liquid produced in step (a) (expressed as sulphur) may be up to 20 grams per litre. Preferably this combined concentration in the loaded aqueous liquid is in the range of from 100 mg/L to 15 g/L, more preferably of from 150 mg/L to 10 g/L. The aqueous liquid may comprise trace compounds, such as for example iron, copper or zinc, as nutrients for the sulphide-oxidising bacteria.

The elemental sulphur particles in the aqueous alkaline liquid of step (a) may suitably be provided by recirculating at least a part of the enriched aqueous liquid of step (b) to step (a). In a particularly preferred embodiment, the bulk of the aqueous alkaline liquid that is provided in step (a) consists of recirculated enriched aqueous liquid from step (b). More preferably, at least 90 vol.% of the alkaline liquid that is provided in step (a) consists of recirculated enriched aqueous liquid from step (b).

In step (b) the loaded aqueous liquid of step (a) is contacted with an oxidant wherein sulphide is oxidised to elemental sulphur by the sulphide-oxidising bacteria. Preferably, the amount of oxidant supplied to the bioreactor in which step (b) may be performed is at least about the stoichiometric amount needed for oxidation of the sulphide in step (a) and/or (b) into elemental sulphur. In this way the protons generated by the bacteria when forming elemental sulphur are consumed thereby regenerating the bacteria. This step is therefore also referred to as a caustic regeneration. The thus obtained regenerated sulphide-oxidising bacteria may subsequently be reused in step (a). Any suitable oxidant may be used, for example nitrate or molecular oxygen, preferably molecular oxygen. The oxidant may be supplied in any suitable way, preferably by supplying a gaseous stream comprising molecular oxygen to a bioreactor. The gaseous stream comprising molecular oxygen may be any suitable gas comprising oxygen, preferably air.

Preferably the temperature in step (b) is in the range of from 10 to 48 °C, more preferably of from 35 to 45 °C and the pressure is between 0 bara and 10 bara, more preferably from atmospheric pressure to 5 bara, even more preferably at atmospheric pressure.

The elemental sulphur particles in the enriched aqueous liquid that is formed in step (b) is separated in step (c). Such a separation may be performed after step (b) has been completed and/or it may be performed simultaneously with step (b). This isolation of elemental sulphur may be performed by any means known in the art, such as for example by means of sedimentation or other means for solid-liquid separation. Preferably elemental sulphur is recovered by taking part of the aqueous solution obtained in step (b) and isolating elemental sulphur from that part to obtain a sulphur-depleted effluent. Part of the sulphur depleted effluent may be recycled to step (b) and part of the sulphur depleted effluent may be purged. Another part of the aqueous solution obtained in step (b) may be used as the aqueous alkaline solution in step (a).

The content of elemental sulphur as part of the polysulphide compounds in the loaded aqueous liquid [$S^0$ in $S_x^{2-}$] is measured according to the following method. The total concentration of polysulphide is first measured spectrophotometrically at a wavelength of 285 nm as described by Kleinjan, W. E.; De Keizer, A.; Janssen, A. J. H., Equilibrium of the reaction between dissolved sodium sulphide and biologically produced sulphur. Colloids and Surfaces B: Biointerfaces 2005,43, (3-4), 228-237. The average chain length is subsequently determined by thermodynamics as described by Alexey Kamyshny, Jenny Gun, Dan Rizkov, Tamara Voitsekovski, and Ovadia Lev in Environ. Sci. Technol. 2007, 41, 7, 2395-2400. The content of elemental sulphur as part of the polysulphide compounds [$S^0$ in $S_x^{2-}$] can now be calculated making use of the measured polysulphide content and the determined average chain length.

The required content of elemental sulphur as part of the polysulphide compounds in the loaded aqueous liquid [$S^0$ in $S_x^{2-}$] or the polysulphidic conditions may be achieved in the process by influencing temperature, residence time and pH or a combination of these measures. Higher sulphide concentration, higher temperature, longer residence time and higher pH favour the formation of polysulphides. Also local high contents of polysulphide and small elemental sulphur particles may further result in a higher polysulphide content at a similar reaction time due to autocatalytic effects. Thus the skilled person may choose various measures to achieve the process conditions of the present invention to perform the process having a good and stable sulphur settlement.

One way of influencing the temperature is wherein the aqueous alkaline liquid is increased in temperature by indirect heat exchange with the loaded aqueous liquid and/or an external heat source thereby obtaining a heated aqueous alkaline liquid which is used in step (a). The loaded aqueous liquid, for example just before it is used in step (b), will suitably have a higher temperature than the loaded aqueous liquid as supplied to step (a). Suitably the loaded aqueous liquid may have a temperature of between 35 and 50 °C. By using this relatively warm stream to increase the temperature of the aqueous alkaline liquid which is to be used in step (a) the required content of elemental sulphur as part of the polysulphide compounds in the loaded aqueous liquid [$S^0$ in $S_x^{2-}$] or the polysulphidic conditions according to this invention may be achieved.

In order to achieve a high content of elemental sulphur as part of the polysulphide compounds in the loaded aqueous liquid as supplied to step (b), the loaded aqueous liquid needs to be given sufficient residence time. By increasing residence time of the loaded aqueous liquid between its preparation in step (a) and its supply to step (b) the formation of elemental sulphur as part of the polysulphide compounds in the loaded aqueous liquid [$S^0$ in $S_x^{2-}$] or the polysulphidic conditions is promoted. This residence time is also referred to as the Sulphidic' Retention Time (SuRT). Preferably, the residence time SuRT is between 3 and 45 minutes, more preferably between 5 and 15 minutes.

The desired residence time is achieved by a process wherein step (a) comprises passing the loaded aqueous liquid flows through a polysulphide reactor zone. In this polysulphide reactor zone polysulphide compounds are formed by reaction of the dissolved sulphide and the elemental sulphur particles. The polysulphide reactor zone comprises one or more plug flow reactor zones. In these plug flow reactor zones back mixing is minimised. The polysulphide reactor zone will as a result have a different content of polysulphide in different regions of the polysulphide reactor zone. This is beneficial because as a result of the autocatalytic nature of the reaction a higher average polysulphide concentration and a higher polysulphide concentration in the effluent of the polysulphide reactor zone is achieved. The term "plug flow reactor zone" as used herein refers to a zone within a tube through which a fluid is flowing, in which zone the velocity of the fluid is substantially constant across any cross-section of the tube perpendicular to the axis of the pipe, assuming that there is no boundary layer adjacent to the inner wall of the tube.

The polysulphide reactor zone may for example be a vertically or horizontally extended vessel having internals along which the liquid flows in a zig-zag flow pattern through the vessel. Such a polysulphide reactor zone will thus have an upstream region and a down stream region. Further the loaded aqueous liquid will have a higher polysulphide content in the downstream region compared to the upstream region.

The required content of elemental sulphur as part of the polysulphide compounds in the loaded aqueous liquid [$S^0$ in $S_x^{2-}$] or the polysulphidic conditions may be achieved in the polysulphide reactor zone by recycling part of the loaded aqueous liquid having a higher polysulphide content from a downstream region of the polysulphide reactor zone to an upstream region in the polysulphide reactor zone where the loaded aqueous liquid has a lower polysulphide content with the object to increase the polysulphide content in the upstream region. It has been found that the presence of polysulphide compounds enhances the formation rate of more polysulphide compounds. Thus by recycling in this way this autocatalytic effect is enhanced resulting in that the conditions of this invention are even more achieved. Suitably between 5 and 50 wt% of the loaded aqueous liquid as discharged at the downstream region is recycled to the upstream region.

Preferably the part of the loaded aqueous liquid having a higher polysulphide content which is recycled is not immediately added to the upstream region in the polysulphide reactor zone, like by means of a conduit and pump. By not immediately adding this part extra residence time for this part will be created thereby allowing that even more polysulphide will form in this part. By adding this polysulphide enriched part to the upstream region in the polysulphide reactor zone an even greater autocatalytic effect will be present. Suitably the part of the loaded aqueous liquid as isolated from the downstream region flows via a zone having a residence time of between 5 and 45 minutes before it is recycled to the upstream region in the polysulphide reactor zone. Preferably the residence time in this zone is between 5 and 15 minutes.

The required content of elemental sulphur as part of the polysulphide compounds in the loaded aqueous liquid [$S^0$ in $S_x^{2-}$] or the polysulphidic conditions may be achieved by directly supplying part of the aqueous alkaline liquid further comprising sulphide-oxidising bacteria to the polysulphide reactor zone. This part thus by-passes the absorption, i.e. the contacting part of step (a) and is directly mixed with the loaded aqueous liquid in the polysulphide reactor zone. Preferably part of the aqueous alkaline liquid is added to the upstream region of the polysulphide reactor zone. Suitably between 5 and 20 wt% of the aqueous alkaline liquid is directly provided to the polysulphide reactor zone while the remaining part is used to contact with the hydrogen sulphide comprising gas.

Increasing the polysulphide content in the part of the loaded aqueous liquid which is recycled to the upstream region in the polysulphide reactor zone may also be achieved by increasing the temperature of this part. Preferably part of the loaded aqueous liquid having a higher polysulphide content is increased in temperature before being recycled to the upstream region in the polysulphide reactor zone. Preferably the temperature is increased to a temperature between 35 and 50 °C.

As also described above, step (a) is preferably performed in a vertical column wherein continuously the hydrogen sulphide comprising gas is fed to the column at a lower position of the column and the aqueous liquid comprising sulphide-oxidising bacteria is continuously fed to a higher position of the column such that a substantially upward flowing gaseous stream contacts a substantially downwards flowing aqueous stream. Preferably part of the aqueous liquid comprising sulphide-oxidising bacteria is continuously fed to a higher position of the column to contact with the upflowing gaseous stream in a first contacting zone which generates an intermediate loaded aqueous liquid. In the first contacting zone the gas is polished to its required low level of hydrogen sulphide. Part of the aqueous liquid comprising sulphide-oxidising bacteria is continuously fed to an intermediate position of the column to contact together with the intermediate loaded aqueous liquid, with the upflowing gaseous stream in a second contacting zone. In this second contacting zone part of the aqueous liquid comprising sulphide-oxidising bacteria contacts fresh feed gas resulting in a higher polysulphide concentration in the combined loaded aqueous liquid. By mixing this loaded aqueous liquid with the intermediate loaded aqueous liquid any small sulphur particles present in the intermediate loaded aqueous liquid will be reacted away by the polysulphides. Preferably between 5 to 50 wt% of the total aqueous liquid comprising sulphide-oxidising bacteria is supplied to this second contacting zone. This embodiment may be performed in a single absorption vessel.

In step (a), in another preferred embodiment, part of the aqueous liquid comprising sulphide-oxidising bacteria is continuously contacted in a step (a1) with the hydrogen sulphide comprising gas to obtain a first intermediate loaded aqueous liquid and an intermediate gas having a lower intermediate content of hydrogen sulphide and another part of the aqueous liquid comprising sulphide-oxidising bacteria is continuously contacted in a step (a2) with the intermediate gas having a lower intermediate content of hydrogen sulphide to obtain a second intermediate loaded aqueous liquid and the gas having a lower content of hydrogen sulphide. Step (a2) may be considered to be a polishing step where the required low level of hydrogen sulphide is achieved. In step (a1) part of the aqueous liquid comprising sulphide-oxidising bacteria contacts fresh feed gas resulting in a higher polysulphide concentration in the first intermediate loaded aqueous liquid. By combining the first intermediate loaded aqueous liquid with the second intermediate loaded aqueous liquid any small sulphur particles present in the second intermediate loaded aqueous liquid will be reacted away by the polysulphides. Preferably between 5 to 50 wt% of the total aqueous liquid comprising sulphide-oxidising bacteria is supplied to step (a1). The first intermediate loaded aqueous liquid is combined with the second intermediate loaded aqueous liquid to obtain the loaded aqueous liquid. The residence time of the first and second intermediate loaded aqueous liquids between step (a) and step (b) is suitably between 5 and 45 minutes and preferably between 5 and 15 minutes.

Preferably each first and second intermediate loaded aqueous liquids produced in step (a1) and (a2) flow through separate first and second polysulphide reactor zones respectively. In the polysulphide reactor zones polysulphide compounds are formed by reaction of the dissolved sulphide and the elemental sulphur. Preferably the polysulphide reactor zones comprise one or more plug flow reactor zones which avoid back mixing as described above. Preferably part of the first intermediate loaded aqueous liquid rich in polysulphides is supplied to the second polysulphide reactor zone to increase the polysulphide content in the second intermediate loaded aqueous liquid. When the polysulphide reactor zones have an upstream and downstream region it is preferred that the part of the first intermediate loaded aqueous liquid rich in polysulphides is supplied to the upstream region of the second polysulphide reactor zone to increase the polysulphide content in the second intermediate loaded aqueous liquid.

Step (a1) and step (a2) may be performed in the same or preferably separate absorption columns. More preferably step (a1) and step (a2) are performed in separate absorption columns, each comprising a lower end where the respective polysulphide reactor zones are present.

The process is suitably performed by making use of a measurement and control where the content of elemental sulphur as part of the polysulphide compounds in the loaded aqueous liquid [$S^0$ in $S_x^{2-}$] or the polysulphidic conditions in the loaded aqueous liquid as supplied to step (b) is measured and when the measured content is below a threshold value the temperature and/or residence time by influencing the recycle is adapted with the object to increase said content as described above.

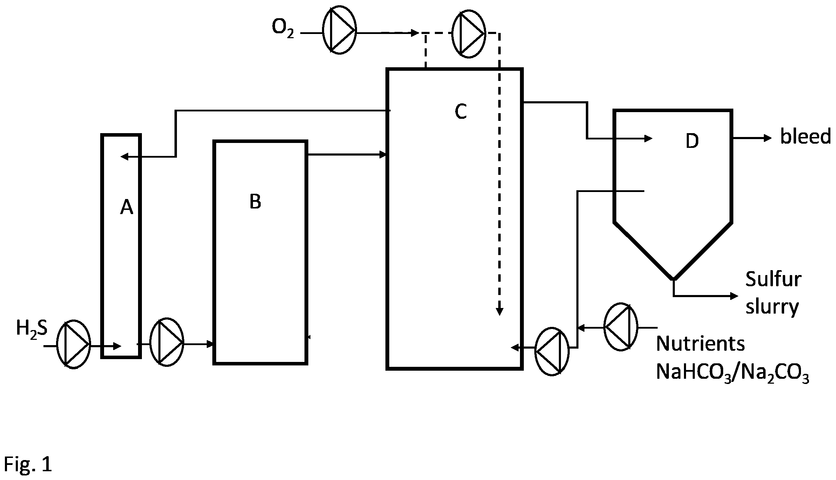

The invention will be illustrated by means of Figures 1-7. Figures 1-4 relate to the Examples.

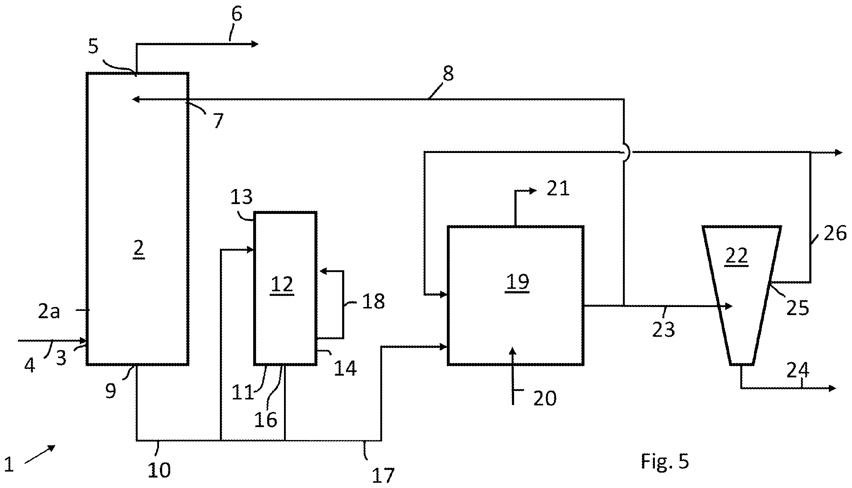

Figure 5 shows a sulphur reclaiming process facility in which the process according to the present invention may be performed. The invention is also directed to this sulphur reclaiming process facility. The sulphur reclaiming process facility (1) is provided with an absorption column (2) provided with an inlet (3) for a hydrogen sulphide comprising gas (4), an outlet (5) for a gas (6) having a lower content of hydrogen sulphide at its upper end, an inlet (7) for an aqueous alkaline liquid (8) further comprising sulphide-oxidising bacteria and a first outlet (9) for a loaded aqueous liquid (10) at a lower elevation. Further a polysulphide reactor zone (11) is shown. This polysulphide reactor zone (11) is part of a separate vessel (12). Alternatively the polysulphide reactor zone (11) may also be positioned in the lower end (2a) of the absorption column (2) or be a combination of these two embodiments. The polysulphide reactor zone (11) comprises a plug flow reactor zone, said polysulphide reactor zone (11) comprising an upstream end (13) and a downstream end (14). The upstream end (13) of the polysulphide reactor zone (11) is fluidly connected to the first outlet (9) for a loaded aqueous liquid (10). The downstream end (14) of the polysulphide reactor zone (11) is provided with a second outlet (16) for the loaded aqueous liquid (17) and with a recycle stream (18) for part of the loaded aqueous liquid to the upstream end (13) of the polysulphide reactor zone (11). This recycle stream (18) achieves that the content of elemental sulphur as part of polysulphide is increased in the loaded aqueous liquid as it is supplied to an aerobic operated bioreactor (19). This content may be increased or decreased by increasing or decreasing the fraction which is recycled. The content of elemental sulphur as part of polysulphide may also be increased by increasing the temperature of the recycle stream (18), by increasing the temperature in the lower end (2a) of the absorption column (2), by increasing the temperature in separate vessel (12) and/or by increasing the time between isolating part of the loaded aqueous fraction from the downstream region (14) and supplying this part at the upstream region (13).

The second outlet (16) for the loaded aqueous liquid (17) is fluidly connected to an aerobic operated bioreactor (19) for performing step (c) of the process. To the aerobic operated bioreactor (19) air (20) is provided and used air (21) is discharged. The aerobic operated bioreactor (19) is fluidly connected to the inlet (7) for the aqueous alkaline liquid (8) of the absorber column (2) and to an elemental sulphur recovery unit (22) via conduit (23). The recovery unit (22) may alternatively be part of bioreactor (19). The elemental sulphur recovery unit (22) is provided with an outlet (24) for elemental sulphur and an outlet (25) for a liquid effluent (26) poor in elemental sulphur. This liquid effluent is partly purged and partly returned to the aerobic operated bioreactor (19) as shown.

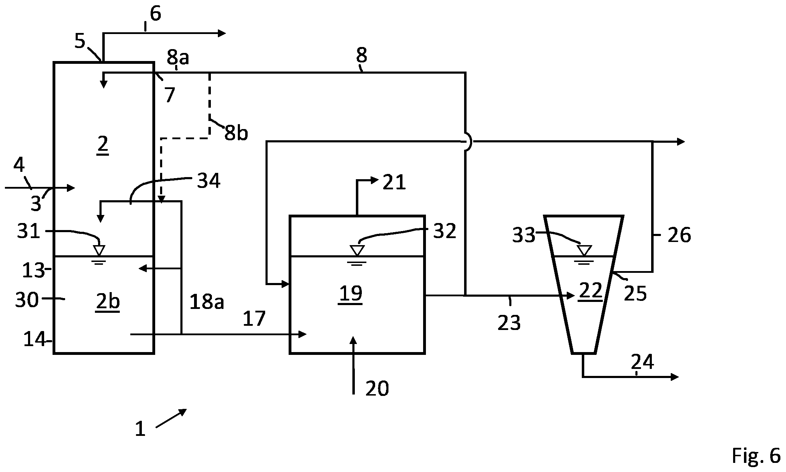

Figure 6 shows a sulphur reclaiming process facility as in Figure 5 with the following differences. Instead of a separate vessel (12) the polysulphide reactor zone (11) is located in the bottom part (2b) of the absorption column (2) as a so-called sump (30). The sump (30) is a volume of loaded aqueous liquid (17) defined at its upper end by a liquid level (31). This liquid level (31) may be at the same elevation as the liquid levels (32) in the aerobic operated bioreactor (19) and the liquid level (33) in the elemental sulphur recovery unit (22) as shown or may be at different elevations. The inlet (3) for a hydrogen sulphide comprising gas (4) is located above the liquid level (31) of sump (30). The sump (30) as the polysulphide reactor zone (11) comprises one or more plug flow reactor zones, said polysulphide reactor zone comprising an upstream end (13) and a downstream end. Part of the loaded aqueous liquid (17) is recycled via recycle stream (18a) to the upstream region (13) of the sump (30). As shown this part may also be supplied to the absorption column (2) at a position (34) above liquid level (31) of sump (30) as recycle stream (18a). This recycle stream (18a) may be combined with part (8b) of the aqueous alkaline liquid (8). Another part (8a) of aqueous alkaline liquid (8) is provided to inlet (7) at the upper end of column (2).

This recycle stream (18a) achieves that the content of elemental sulphur as part of polysulphide is increased in the loaded aqueous liquid (17) before it is supplied to an aerobic operated bioreactor (19). This content may be increased or decreased by increasing or decreasing the fraction (18a) which is recycled. The content of elemental sulphur as part of polysulphide may also be increased by increasing the temperature of the recycle stream (18a), by increasing the temperature in sump (30) and/or by increasing the time between isolating part of the loaded aqueous fraction from the downstream region (14) and supplying this part at the upstream region (13) or to position (34).

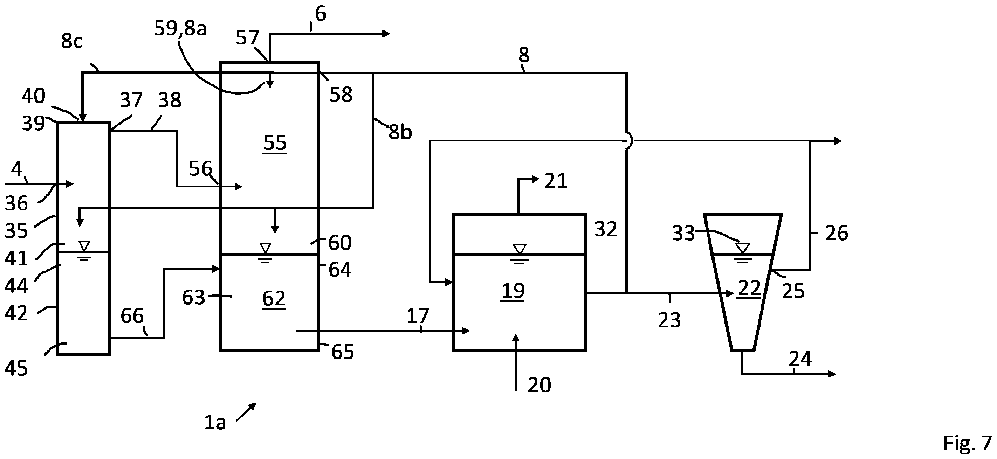

Figure 7 shows a sulphur a reclaiming process facility (1a) comprising a first absorption column (35) provided with an inlet (36) for a hydrogen sulphide comprising gas (4), an outlet (37) for an intermediate gas (38) having a lower content of hydrogen sulphide at its upper end (39), an inlet (40) for part (8c) of an aqueous alkaline liquid (8) further comprising sulphide-oxidising bacteria and an outlet (41) for a first intermediate loaded aqueous liquid at a lower elevation. Also a second absorption column (55) is shown provided with an inlet (56) for the intermediate gas (38) having a lower content of hydrogen sulphide, an outlet (57) for a gas (6) having a lower content of hydrogen sulphide at its upper end (58), an inlet (59) for part (8a) of an aqueous alkaline liquid (8) further comprising sulphide-oxidising bacteria and a outlet (60) for a second intermediate loaded aqueous liquid at a lower elevation.

A polysulphide reactor zone (42) is part of the first absorption column (35) and positioned in the lower end (43) of the first absorption column (35). A polysulphide reactor zone (62) is part of the second absorption column (55) and positioned in the lower end (63) of the second absorption column (55). The polysulphide reactor zones (42,62) comprise one or more plug flow reactor zones, said sulphide reactor zones (42,62) comprising an upstream end (44,64) and a down stream end (45,65).

The upstream end (44) of the polysulphide reactor zone (42) of the first absorption column (35) is fluidly connected to the outlet (41) for the first intermediate loaded aqueous liquid and the upstream end (64) of the polysulphide reactor zone (62) of the second absorption column (55) is fluidly connected to the outlet (60) for the second intermediate loaded aqueous liquid. The downstream end (45) of the polysulphide reactor zone (42) of the first absorption column (35) is fluidly connected to the upstream end (64) of the polysulphide reactor zone (62) of the second absorption column (55) via stream (66). In this manner a fraction comprising high contents of polysulphide are added to the polysulphide reactor zone (62). The resulting loaded aqueous liquid (17) will then have the claimed properties. This loaded aqueous liquid (17) is supplied to an aerobic operated bioreactor (19) for oxidation of sulphide to elemental sulphur. For this the downstream end (65) of the polysulphide reactor zone (62) of the second absorption column (55) is fluidly connected to an aerobic operated bioreactor (19) for regeneration of the sulphide-oxidising bacteria.

Part of the contents of the polysulphide reactor zone (42) of the first absorption column (35) may be directly supplied to bioreactor (19) (not shown). Part (8b) of the aqueous alkaline liquid (8) further comprising sulphide-oxidising bacteria is added to first and second absorber columns (35,55) to further enhance the formation of polysulphides. The first absorber column may have a simple design, not necessarily provided with contacting internals. Step (a1) described above may be performed in the first absorber column (35). Second absorber column (55) is suitably provided with contacting internals such to optimise the gas-liquid contacting such to achieve an optimal absorption of the hydrogen sulphide. Step (a2) described above may be performed in the second absorber column (55).

The aerobic operated bioreactor (19) is fluidly connected to the inlet (59) for a part (8a) of the aqueous alkaline liquid (8) of the second absorber column and fluidly connected to the inlet (40) for a part (8c) of the aqueous alkaline liquid (8) of the first absorber column. The elemental sulphur recovery unit (22) is provided with an inlet fluidly connected to the aerobic operated bioreactor (19) and provided with an outlet (24) for elemental sulphur and an outlet (25) for a liquid effluent poor in elemental sulphur.

The polysulphide reactor zone (11,2b, 42,62) of Figures 5-7 may be provided with means to increase the temperature of the liquid contents of the polysulphide reactor zone (11,2a, 42,62). This may be by indirect heat exchange wherein for example a hot heating medium flows through tubes as present in the polysulphide reactor zone (11,2b, 42,62) thereby heating up the liquid contents of these zones.

The invention will be illustrated by the following non-limiting experiments.

Examples

Here we report the effects of a novel sulphidic reactor inserted in the conventional process set-up. A sulphidic reactor is defined as conditions where dissolved oxygen is below 1 $\mu M$ $O_2$ and sulphides are above 0.5 mM. We analyzed sulfur particles produced in continuous, long term lab-scale reactor experiments under various sulphide concentrations and sulphidic retention times. The analysis was performed with laser diffraction particle size analysis and light microscopy

Two identical lab-scale reactor set-ups were used with an absorber (A) having a liquid volume of 0.4 L and microaerophilic gas-lift reactor (C) having a liquid volume of 3.7 L ( as shown in Fig.1). Two additional reactor compartments could be added: a polysulphide reactor zone (B) having a liquid volume of 3.5 L between the absorber (A) and the microaerophilic gas-lift reactor (C) and a settler (D) having a liquid volume of 1.5 L after the microaerophilic gas-lift reactor.

A settler was included for the experiments with the highest $H_2S$ loading rate to prevent sulphur accumulation in the system, i.e. to avoid operational issues such as foaming and clogging due to sulphur build-up. Experiments with lower $H_2S$ loading rate were conducted without settler to collect a sample in which all particles were present that were produced under the specific experimental conditions, without removing any particles with the settler. The polysulphide reactor zone (B) is a zone with a retention time of reactor content (medium, microorganisms and sulphur particles) under anaerobic, (poly)sulphidic pressure. The presence of the polysulphide reactor zone (B) increases the Sulphidic' Retention Time (SuRT).

The experiments carried out with the various conditions are numbered Examples 1-3 and Comparative Experiment A. An overview of the operational conditions per experiment is described in Table 1. The gas flow was recycled over the headspace of microaerophilic gas-lift reactor with a vacuum pump to prevent any release of $H_2S$ gas and to reach low oxygen concentrations. The gas was introduced with a porous stone to the bottom of the inner column of the microaerophilic gas-lift reactor (C) to ensure proper oxygen transfer and mixing. Pure $H_2S$ gas and oxygen were supplied by mass flow controllers. In case of pressure build-up, excess gas was discharged via a water-lock saturated with zinc acetate to capture any potentially present $H_2S$. The reactors were operated at 35 °C using a thermostat bath and climate-controlled cabinet.

Table 1

| Process condition | Comparative Experiment A | Example 1 | Example 2 | Example 3 |
|---|---|---|---|---|
| Polysulphidic reactor zone (B) | no | yes | yes | yes |
| Settler (D) | Yes | Yes | No | No |
| SuRT (min) | 4.8 | 45.0 | 38.7 | 38.7 |
| Total dissolved sulphide in polysulphidic reactor zone (mM) | 8.3 | 4.4 | 1.9 | 1.0 |
| S-loading rate (g S L-1 microaerophilic reactor day-1) | 4.4 | 4.4 | 2.0 | 1.0 |
| Total system volume a (L) | 5.6 | 9.1 | 7.6 | 7.6 |
| pH in reactor (C) | 8.4 | 8.5 | 8.7 | 8.5 |
| Duration (days) | 31 | 28 | 16 | 41 |

The medium consisted of a buffer with 6.6 g L$^{-1}$ $Na_2CO_3$ and 69.3 g L$^{-1}$ $NaHCO_3$ in demineralized water at pH 8.5. Fresh buffer was supplied at a constant flow to maintain enough alkalinity in the system. Furthermore, a nutrient stock was supplied for biological growth containing (in g per 1 L of demineralized water): $K_2HPO_4$, 0.1; $MgCl_2$ $6H_2O$, 0.0203; NaCl, 0.6; $CH_4N_2O$, 0.06 and 2 mL L$^{-1}$ trace element solution as in Pfennig, N., Lippert, K.D., 1966. Uber das Vitamin B12-bedurfnis phototropher Schwefelbacterien. Arch. Microbiol. 55, 245-256.

Comparative Experiment A was inoculated with centrifuged microorganisms (to remove excess sulfur) from a lab-scale sulfur producing gas-lift bioreactor, operated under continuous conditions, like the conditions applied in these experiments. The original inoculum of this reactor was obtained from a well-characterized industrial scale Thiopaq process of applicant. To remove the sulfur, the reactor content was centrifuged at 4500 RPM for 20 minutes (using a FirLabO, Froilabo, Paris, France). A pellet was formed with two layers: a bottom layer of elemental sulfur and a pellet with microorganisms on top. The pellet with microorganisms was carefully washed off. Example 3 was inoculated with centrifuged microorganisms directly taken from the above mentioned Thiopaq process. Experiment 1 and 2 were inoculated with microorganism-rich process solution from Comparative Experiment A and Example 3.

Reactors (B,C) were filled with medium and inoculated. In all experiments, the set-up was operated in continuous mode without interruption. Throughout all experiments, the $H_2S$ load was kept constant for that experiment. The $H_2S$ load was used to set the total sulphide concentration in the polysulphide reactor zone . To keep the conversion efficiency from sulphide to sulfur high, the oxidation-reduction potential (ORP) was set at -360 mV vs. Ag/AgCl, which is a representative set-point for industrial reactors. The ORP set-point was controlled by a proportional-integral (PI) controller. The PI controller regulated the oxygen supply rate. Samples (well-mixed reactor content with sulfur particles, medium and micro-organisms) were taken for analysis at a sampling port in the middle of the polysulphide reactor zone (B) (Exp. 2 and 3) and the microaerophilic gas-lift reactor (C) (all experiments). The sampling tubes from the reactor were flushed three times prior to sampling to obtain a representative sample.

Reactors were equipped with sensors for temperature and ORP (Triple Junction, platinum rod, glass electrode equipped with an internal Ag/AgCl reference electrode, ProSense, Oosterhout, The Netherlands). The particle size distribution (PSD) is expressed both volumetrically and numerically; in a volumetric based particle size distribution, larger particles have a heavier weight as, due to their size, they often comprise a larger percentage of the total solid volume. In a numeric based distribution, each particle has an equal weight, independent of the particle size. According to common practice, when a PSD must be represented by a single value, the median (D50) of the PSD was reported to show the particle size development over time. The median has a better way of representing the central location of the data in a non-normal distribution than the mean.

The process selectivity for elemental sulfur was calculated by the mass balance based on the $H_2S$ supply and measurement of dissolved sulfur products formed. The term 'HS-' is used to refer to the sum of total dissolved sulphide ($H_2S$, HS- and S$^{2-}$) as most of the dissolved sulphide is present as HS- at pH 8.5.

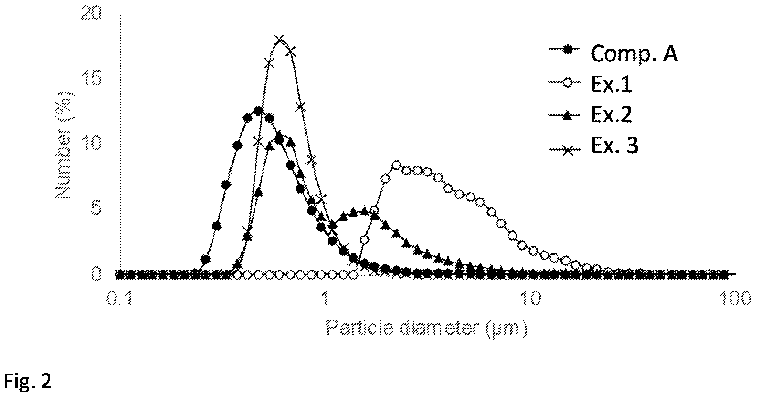

In Experiment A and in Examples 1, 2 and 3 sulphide was successfully converted to elemental sulfur, leading to the presence of sulfur particles in the reactor solution. Typical particle size distributions (PSD) of sulfur particle samples taken from the microaerophilic reactor during these experiments are shown in Figure 2.

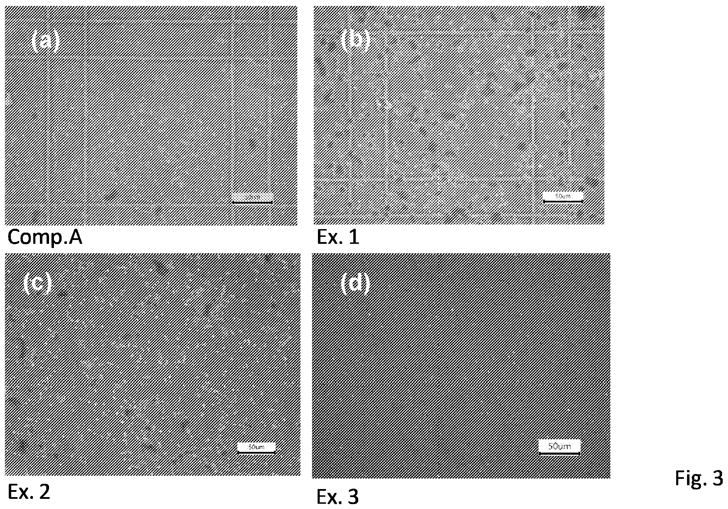

The sulfur particles formed under the various experimental conditions had distinctively different morphologies as observed with light microscopy as shown in Figure 3. Sulfur particles can be distinguished in the light microscopy pictures as light, radiant particles (single particles) or darker patches with light, radiant edges (agglomerated particles). The black spots in the background are microorganisms.

In the pictures of comparative Experiment A many small individual (sub)micron-sized sulfur particles are visible, which is in good agreement with the particle size distribution shown in Figure 2. In Comparative Experiment A also large agglomerates (~20 $\mu$m) are present in seemingly low concentration. The concentration was likely too low to be visible in the number-based particle size distribution. In Comparative Experiment A the particles seem mainly globular (rough and smooth) and around a size of 1 $\mu$m.

In Example 1, however, small (sub)micron particles are hardly visible (Fig. 3(b)). Also, in Example 2 not a lot of these particles seem present, at least not as many as in Comparative Experiment A (Fig. 3(c)). Larger, agglomerated, sulfur particles can be observed in the pictures of Examples 1 and 2. In both examples, large agglomerates are visible of 20-30 $\mu$m, but also smaller ones of 5-10 $\mu$m. The center of the agglomerates appears dark due to the thickness of the sample, which could be observed while focusing the microscope. No microorganisms were observed attached to the agglomerates, as they could easily be distinguished as small black spots of around 1 $\mu$m in the sample. It is possible that due to mixing during PSD measurement, the larger aggregates were slightly broken down, and thus not measured. In addition, since the PSD is number based, the small particles weigh just as heavy in the distribution as the larger ones, and there are clearly more small particles than larger ones in terms of numbers. However, the larger ones are more visible in the microscopy picture. From the rough, lumpy edges of the agglomerates it can be observed that they are composed of many small constituent crystals.

Figure 3 shows the influence of a polysulphide reactor zone on the formation of agglomerates. Further the light microscopy pictures show the smallest particles were hardly present in Example 1 and to only some extent in Examples 2 and 3.

The removal of the smallest particles in Examples 1, 2 and 3 is related to the formation of polysulphides in the polysulphide reactor zone. Polysulphides are yellow to orange and by the yellow color of the sulphidic reactor, it could be deduced that indeed polysulphides were formed.

The bisulphide content, polysulphide content, average chain length and the content of elemental sulphur as part of polysulphide was measured according to the method of this invention. It was found that these measurements fitted well to a mathematical model. The model inputs are the volume based average PSD of the four experiments and the operational conditions under which these particles were produced. From these PSDs, the volume fraction of particles with a diameter <1 $\mu$m was calculated. By multiplying this volume fraction with the average measured concentration of elemental sulfur in the experiments, the total concentration of particles <1 $\mu$m was calculated. Then, three outputs were calculated: the percentage of elemental sulphur ($S_0$) in that could be converted to polysulphide ($S_x^{2-}$), the percentage equilibrium $S_x^{2-}$ and the absolute content of elemental sulphur as part of polysulphide ($S_0$ in $S_x^{2-}$), expressed in mM.

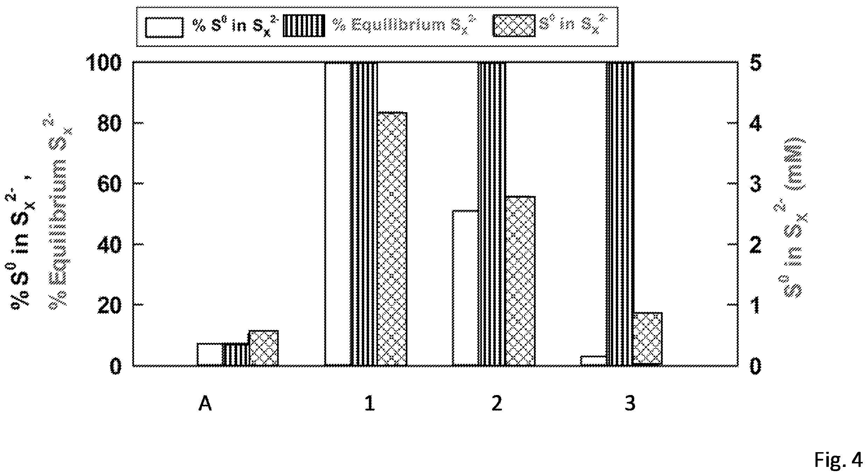

Our modelling results support the experimentally obtained findings that the smallest sulfur particles dissolve in the polysulphide reactor zone, due to polysulphide formation to the extent that the conditions allowed for. Equilibrium $S_x^{2-}$ between sulfur, sulphide and polysulphide was reached for Examples 1, 2 and 3 (See Figure 4). In Comparative Experiment A little polysulphide formation was expected due to the short SuRT, which agrees with the large amount of submicron particles found in the corresponding lab experiment. The modelling results support this. These particles would have been the ones most prone to react to polysulphide, due to their high surface-to-volume ratio.

These results from these experiments and models illustrate the invention: when the sulfur absorbing column is provided with reactors that promote the correct degree of mixing and residence time, and/or with higher starting sulphide concentrations, the higher (or total) equilibrium achieved between polysulphides and sulphides allow for the reacting-away of small elemental sulfur particles that were provided to the sulphidic chamber to form polysulphides. The resulting steady-state in the system (for example as measured in the bioreactor) is absent of smaller sulfur particles (< 1 $\mu$m), and/or is concentrated in larger particles.

**Claims**

1. A process to continuously treat a hydrogen sulphide comprising gas, said process comprising the following steps:

    (a) contacting the hydrogen sulphide comprising gas with an aqueous alkaline liquid comprising sulphide-oxidising bacteria and elemental sulphur particles thereby producing a loaded aqueous liquid comprising dissolved sulphide, polysulphide compounds, sulphide-oxidising bacteria and elemental sulphur particles and a gas having a lower content of hydrogen sulphide, and passing the loaded aqueous liquid through a polysulphide reactor zone comprising one or more plug flow reactor zones,
    (b) contacting the loaded aqueous liquid with an oxidant to enable the sulphide-oxidising bacteria to oxidise sulphide to elemental sulphur, thereby producing an enriched aqueous liquid comprising an increased amount of elemental sulphur particles, and
    (c) separating elemental sulphur particles from the enriched aqueous liquid,

wherein the residence time of the loaded aqueous liquid between its preparation in step (a) and its supply to step (b) is between 3 and 45 minutes, and

wherein the content of elemental sulphur as part of the polysulphide compounds in the loaded aqueous liquid [$S^0$ in $S_x^{2-}$] as supplied to step (b) is above 0.7 mM, preferably above 1 mM.

2. Process according to claim 1, wherein for a period of at least 1 week the daily average content of elemental sulphur as part of the polysulphide compounds in the loaded aqueous liquid [$S^0$ in $S_x^{2-}$] as supplied to step (b) is above 0.7 mM.

3. Process according to claim 1 or 2, wherein the content of elemental sulphur as part of the polysulphide compounds in the loaded aqueous liquid meets the following condition:

$$\left[S^0 \text{ in } S_x^{2-}\right] \geq 1.7 * \left[HS^-\right] * 10^{(-9.17+pH)} \, ,$$

wherein [$S^0$ in $S_x^{2-}$] is the content of elemental sulphur as part of the polysulphide compounds in the loaded aqueous liquid as supplied to step (b) expressed in mM, [$HS^-$] is the sulphide concentration expressed in mM of the loaded aqueous liquid as supplied to step (b), and wherein pH is the pH of the loaded aqueous liquid as supplied to step (b).

4. Process according to any one of claims 1-3, wherein the hydrogen sulphide comprising gas has a hydrogen sulphide content of between 0.1 and 3 vol.% and a carbon dioxide content of above 20 vol%.

5. Process according to any one of claims 1-4, wherein step (a) comprises passing the loaded aqueous liquid through a polysulfide reactor zone comprising one or more plug flow reactor zones, the polysulphide reactor zone having an upstream region and a downstream region, and wherein preferably in the polysulphide reactor zone part of the loaded aqueous liquid is recycled from the downstream region to the upstream region in the polysulphide reactor zone.

6. Process according to any one of claims 1-5, wherein step (a) is performed in a vertical column wherein continuously the hydrogen sulphide comprising gas is fed to the column at a lower position of the column and the aqueous liquid comprising sulphide-oxidising bacteria is continuously fed to a higher position of the column such that a substantially upward flowing gaseous stream contacts a substantially downwards flowing aqueous stream.

7. Process according to any one of claims 1-3, wherein as part of step (a) part of the aqueous liquid comprising sulphide-oxidising bacteria is continuously contacted in a step (a1) with the hydrogen sulphide comprising gas to obtain a first intermediate loaded aqueous liquid and an intermediate gas having a lower intermediate content of hydrogen sulphide,

wherein as part of step (a) another part of the aqueous liquid comprising sulphide-oxidising bacteria is continuously contacted in a step (a2) with the intermediate gas having a lower intermediate content of hydrogen sulphide to obtain a second intermediate loaded aqueous liquid and the gas having a lower content of hydrogen sulphide, and

wherein the first intermediate loaded aqueous liquid is combined with the second intermediate loaded aqueous liquid to obtain the loaded aqueous liquid.

8. Process according to claim 7, wherein each first and second intermediate loaded aqueous liquids flow through separate first and second polysulphide reactor zones respectively in which polysulphide reactor zones polysulphide compounds are formed by reaction of the dissolved sulphide and the elemental sulphur.

9. Process according to any one of claims 1-8, wherein at least a part of the enriched aqueous liquid of step (b) is recirculated to step (a).

10. A sulphur reclaiming process facility (1) comprising:

- an absorption column (2) provided with an inlet (3) for a hydrogen sulphide comprising gas (4), an outlet (5) for a gas (6) having a lower content of hydrogen sulphide at its upper end, an inlet (7) for an aqueous alkaline liquid (8) further comprising sulphide-oxidising bacteria and a first outlet (9) for a loaded aqueous liquid (10) at a lower elevation,

- a polysulphide reactor zone (11) as part of the absorption column (2) and positioned in the lower end (2a) of the absorption column (2) and/or as part of a separate vessel,

wherein the polysulphide reactor zone (11) comprises one or more plug flow reactor zones, said polysulphide reactor zone (11) comprising an upstream end (13) and a downstream end (14),

wherein the upstream end (13) of the polysulphide reactor zone (11) is fluidly connected to the first outlet (9) for a loaded aqueous liquid (10),

wherein the downstream end (14) of the polysulphide reactor zone (11) is provided with a second outlet (16) for the loaded aqueous liquid (17) and with a recycle stream (18) for part of the loaded aqueous liquid to the upstream end (13) of the polysulphide reactor zone (11), said recycle stream preferably comprising a vessel for increasing the residence time in the recycle,

wherein the second outlet (16) for the loaded aqueous liquid (17) is fluidly connected to an aerobic bioreactor (19) for oxidation of sulphide to elemental sulphur,

wherein the aerobic bioreactor (19) is fluidly connected to the inlet (7) for an aqueous alkaline liquid (8) of the absorber column (2),

and comprising an elemental sulphur recovery unit (22) provided with an inlet fluidly connected to the aerobic bioreactor (19) and provided with an outlet (24) for elemental sulphur and an outlet (25) for a liquid effluent poor in elemental sulphur.

11. A sulphur reclaiming process facility (1a) comprising:

- a first absorption column (35) provided with an inlet (36) for a hydrogen sulphide comprising gas (4), an outlet (37) for an intermediate gas (38) having a lower content of hydrogen sulphide at its upper end (39), an inlet (40) for part (8c) of an aqueous alkaline liquid (8) further comprising sulphide-oxidising bacteria and an outlet (41) for a first intermediate loaded aqueous liquid at a lower elevation,

- a second absorption column (55) provided with an inlet (56) for the intermediate gas (38) having a lower content of hydrogen sulphide, an outlet (57) for a gas (6) having a lower content of hydrogen sulphide at its upper end (58), an inlet (59) for part (8a) of an aqueous alkaline liquid (8) further comprising sulphide-oxidising bacteria and a outlet (60) for a second intermediate loaded aqueous liquid at a lower elevation,

- a polysulphide reactor zone (42) as part of the first absorption column (35) and positioned in the lower end (43) of the first absorption column (35) and/or as part of a separate vessel,

- a polysulphide reactor zone (62) as part of the second absorption column (55) and positioned in the lower end (63) of the second absorption column (55) and/or as part of a separate vessel,

wherein the polysulphide reactor zones (42,62) comprise plug flow zones, said sulphide reactor zones (42,62) comprising an upstream end (44,64) and a downstream end (45,65),

wherein the upstream end (44) of the polysulphide reactor zone (42) of the first absorption column (35) is fluidly connected to the outlet (41) for the first intermediate loaded aqueous liquid and the upstream end (64) of the polysulphide reactor zone (62) of the second absorption column (55) is fluidly connected to the outlet (60) for the second intermediate loaded aqueous liquid,

wherein the downstream end (45) of the polysulphide reactor zone (42) of the first absorption column (35) is fluidly connected to the upstream end (64) of the polysulphide reactor zone (62) of the second absorption column (55),

wherein the downstream end (65) of the polysulphide reactor zone (62) of the second absorption column (55) is fluidly connected to an aerobic bioreactor (19) for oxidation of sulphide to elemental sulphur,

wherein the aerobic bioreactor (19) is fluidly connected to the inlet (59) for a part (8a) of the aqueous alkaline liquid (8) of the first absorber column and fluidly connected to the inlet (40) for a part (8c) of the aqueous alkaline liquid (8) of the second absorber column, and

comprising an elemental sulphur recovery unit (22) provided with an inlet fluidly connected to the aerobic bioreactor (19) and provided with an outlet (24) for elemental sulphur and an outlet (25) for a liquid effluent poor in elemental sulphur.

12. Sulphur reclaiming process facility according to claim 10 or 11, wherein the polysulphide reactor zone (11,42,62) is provided with means to increase the temperature of the liquid contents of the polysulphide reactor zone (11,42,62).

13. Sulphur reclaiming process facility according to any one of claims 10-12 comprising sulphide-oxidising bacteria.

14. Process according to claim 6 as performed in a sulphur reclaiming process facility according to claim 10.

15. Process according to claim 7 as performed in a sulphur reclaiming process facility according to claim 11.

**Patentansprüche**

1. Verfahren zur kontinuierlichen Behandlung eines Schwefelwasserstoff enthaltenden Gases, wobei das Verfahren die folgenden Schritte umfasst:

    (a) Inkontaktbringen des Schwefelwasserstoff enthaltenden Gases mit einer wässrigen alkalischen Flüssigkeit, die sulfidoxidierende Bakterien und elementare Schwefelteilchen enthält, wodurch eine beladene wässrige Flüssigkeit erzeugt wird, die gelöstes Sulfid, Polysulfidverbindungen, sulfidoxidierende Bakterien und elementare Schwefelteilchen und ein Gas mit einem geringeren Gehalt an Schwefelwasserstoff umfasst, und Durchleiten der beladenen wässrigen Flüssigkeit durch eine Polysulfidreaktorzone, die eine oder mehrere Pfropfenströmungsreaktorzonen umfasst,
    (b) Inkontaktbringen der beladenen wässrigen Flüssigkeit mit einem Oxidationsmittel, um die sulfidoxidierenden Bakterien in die Lage zu versetzen, Sulfid zu elementarem Schwefel zu oxidieren, wodurch eine angereicherte wässrige Flüssigkeit erzeugt wird, die eine erhöhte Menge an elementaren Schwefelteilchen enthält, und
    (c) Abtrennen von elementaren Schwefelpartikeln aus der angereicherten wässrigen Flüssigkeit, wobei die Verweilzeit der beladenen wässrigen Flüssigkeit zwischen ihrer Zubereitung in Schritt (a) und ihrer Zuführung zu Schritt (b) zwischen 3 und 45 Minuten beträgt, und

    wobei der Gehalt an elementarem Schwefel als Teil der Polysulfidverbindungen in der beladenen wässrigen Flüssigkeit [$S^0$ in $S_x^{2-}$], wie sie Schritt (b) zugeführt wird, über 0,7 mM, vorzugsweise über 1 mM liegt.

2. Verfahren nach Anspruch 1, wobei über einen Zeitraum von mindestens einer Woche der tägliche Durchschnittsgehalt an elementarem Schwefel als Teil der Polysulfidverbindungen in der beladenen wässrigen Flüssigkeit [$S^0$ in $S_x^{2-}$], wie sie Schritt (b) zugeführt wird, über 0,7 mM liegt.

3. Verfahren nach Anspruch 1 oder 2, wobei der Gehalt an elementarem Schwefel als Teil der Polysulfidverbindungen in der beladenen wässrigen Flüssigkeit die folgende Bedingung erfüllt:

$$\left[ S^0 \text{ in } S_x^{2-} \right] \geq 1.7 * [HS^-] * 10^{(-9,17+pH)} \, ,$$

    wobei [$S^0$ in $S_x^{2-}$] der Gehalt an elementarem Schwefel als Teil der Polysulfidverbindungen in der beladenen wässrigen Flüssigkeit, wie sie Schritt (b) zugeführt wird, ausgedrückt in mM, ist, [$HS^-$] ist die Sulfidkonzentration, ausgedrückt in mM, der beladenen wässrigen Flüssigkeit, wie sie Schritt (b) zugeführt wird, und wobei pH der pH der beladenen wässrigen Flüssigkeit ist, wie sie Schritt (b) zugeführt wird.

4. Verfahren nach einem der Ansprüche 1 bis 3, wobei das Schwefelwasserstoff enthaltende Gas einen Schwefelwasserstoffgehalt zwischen 0,1 und 3 Vol.-% und einen Kohlendioxidgehalt von über 20 Vol-% aufweist.

5. Verfahren nach einem der Ansprüche 1 bis 4, wobei Schritt (a) das Durchleiten der beladenen wässrigen Flüssigkeit durch eine Polysulfidreaktorzone umfasst, die eine oder mehrere Pfropfenströmungsreaktorzonen umfasst, wobei die Polysulfidreaktorzone einen stromaufwärts gelegenen Bereich und einen stromabwärts gelegenen Bereich aufweist, und wobei vorzugsweise in der Polysulfidreaktorzone ein Teil der beladenen wässrigen Flüssigkeit aus dem stromabwärts gelegenen Bereich in den stromaufwärts gelegenen Bereich in der Polysulfidreaktorzone zurückgeführt wird.

6. Verfahren nach einem der Ansprüche 1 bis 5, wobei Schritt (a) in einer vertikalen Kolonne durchgeführt wird, wobei kontinuierlich das Schwefelwasserstoff enthaltende Gas an einer unteren Position der Kolonne in die Kolonne eingespeist wird und die wässrige Flüssigkeit, die sulfidoxidierende Bakterien enthält, kontinuierlich in eine höhere Position der Kolonne eingespeist wird, so dass ein im Wesentlichen aufwärts fließender Gasstrom einen im Wesentlichen abwärts fließenden wässrigen Strom berührt.

7. Verfahren nach einem der Ansprüche 1 bis 3, wobei als Teil des Schritts (a) ein Teil der wässrigen Flüssigkeit, die sulfidoxidierende Bakterien enthält, in einem Schritt (a1) kontinuierlich mit dem Schwefelwasserstoff enthaltenden Gas in Kontakt gebracht wird, um eine erste beladene wässrige Zwischenflüssigkeit und ein Zwischengas mit einem niedrigeren Zwischengehalt an Schwefelwasserstoff zu erhalten,

    wobei als Teil von Schritt (a) ein anderer Teil der wässrigen Flüssigkeit, die sulfidoxidierende Bakterien enthält, kontinuierlich in einem Schritt (a2) mit dem Zwischengas mit einem niedrigeren Zwischengehalt an Schwefelwasserstoff in Kontakt gebracht wird, um eine zweite beladene wässrige Zwischenflüssigkeit und das Gas mit einem niedrigeren Gehalt an Schwefelwasserstoff zu erhalten, und

wobei die erste beladene wässrige Zwischenflüssigkeit mit der zweiten beladenen wässrigen Zwischenflüssigkeit kombiniert wird, um die beladene wässrige Flüssigkeit zu erhalten.

8. Verfahren nach Anspruch 7, bei dem jede erste und zweite beladene wässrige Zwischenflüssigkeit durch getrennte erste bzw. zweite Polysulfidreaktorzonen fließt, wobei in den Polysulfidreaktorzonen Polysulfidverbindungen durch Reaktion des gelösten Sulfids und des elementaren Schwefels gebildet werden.

9. Verfahren nach einem der Ansprüche 1-8, wobei zumindest ein Teil der angereicherten wässrigen Flüssigkeit aus Schritt (b) in Schritt (a) zurückgeführt wird.

10. Anlage (1) zur Rückgewinnung von Schwefel, umfassend:

- eine Absorptionskolonne (2), die mit einem Einlass (3) für ein Schwefelwasserstoffenthaltendes Gas (4), einem Auslass (5) für ein Gas (6) mit einem geringeren Gehalt an Schwefelwasserstoff an ihrem oberen Ende, einem Einlass (7) für eine wässrige alkalische Flüssigkeit (8), die ferner sulfidoxidierende Bakterien enthält, und einem ersten Auslass (9) für eine beladene wässrige Flüssigkeit (10) in einer unteren Höhe versehen ist,

- eine Polysulfidreaktorzone (11) als Teil der Absorptionskolonne (2), die am unteren Ende (2a) der Absorptionskolonne (2) und/oder als Teil eines separaten Behälters angeordnet ist,

wobei die Polysulfidreaktorzone (11) eine oder mehrere Pfropfenströmungsreaktorzonen umfasst, wobei die Polysulfidreaktorzone (11) ein stromaufwärts gelegenes Ende (13) und einem stromabwärts gelegenenes Ende (14) umfasst,

wobei das stromaufwärts gelegene Ende (13) der Polysulfidreaktorzone (11) mit dem ersten Auslass (9) für eine beladene wässrige Flüssigkeit (10) fluidleitend verbunden ist,

wobei das stromabwärts gelegene Ende (14) der Polysulfidreaktorzone (11) mit einem zweiten Auslass (16) für die beladene wässrige Flüssigkeit (17) und mit einem Rückführstrom (18) für einen Teil der beladenen wässrigen Flüssigkeit zum stromaufwärts gelegenen Ende (13) der Polysulfidreaktorzone (11) versehen ist, wobei der Rückführstrom vorzugsweise einen Behälter zur Erhöhung der Verweilzeit im Rücklauf umfasst,

wobei der zweite Auslass (16) für die beladene wässrige Flüssigkeit (17) mit einem aeroben Bioreaktor (19) zur Oxidation von Sulfid zu elementarem Schwefel fluidleitend verbunden ist,

wobei der aerobe Bioreaktor (19) mit dem Einlass (7) für eine wässrige alkalische Flüssigkeit (8) der Absorptionskolonne (2) fluidleitend verbunden ist,

und eine Einheit (22) zur Rückgewinnung von elementarem Schwefel umfasst, die mit einem Einlass versehen ist, der mit dem aeroben Bioreaktor (19) fluidleitend verbunden ist, und die mit einem Auslass (24) für elementaren Schwefel und einem Auslass (25) für einen an elementarem Schwefel armen flüssigen Abfluss versehen ist.

11. Anlage zur Rückgewinnung von Schwefel (1a), umfassend:

- eine erste Absorptionskolonne (35), die mit einem Einlass (36) für ein Schwefelwasserstoff enthaltendes Gas (4), einem Auslass (37) für ein Zwischengas (38), das an seinem oberen Ende (39) einen geringeren Gehalt an Schwefelwasserstoff aufweist, einem Einlass (40) für einen Teil (8c) einer wässrigen alkalischen Flüssigkeit (8), die ferner sulfidoxidierende Bakterien enthält, und einem Auslass (41) für eine erste beladene wässrige Zwischenflüssigkeit in einer niedrigeren Höhe versehen ist,

- eine zweite Absorptionskolonne (55), die mit einem Einlass (56) für das Zwischengas (38) mit einem geringeren Gehalt an Schwefelwasserstoff, einem Auslass (57) für ein Gas (6) mit einem geringeren Gehalt an Schwefelwasserstoff an ihrem oberen Ende (58), einem Einlass (59) für einen Teil (8a) einer wässrigen alkalischen Flüssigkeit (8), die ferner sulfidoxidierende Bakterien enthält, und einem Auslass (60) für eine zweite beladene wässrige Zwischenflüssigkeit in einer niedrigeren Höhe versehen ist,

- eine Polysulfidreaktorzone (42) als Teil der ersten Absorptionskolonne (35) und angeordnet im unteren Ende (43) der ersten Absorptionskolonne (35) und/oder als Teil eines separaten Behälters,

- eine Polysulfidreaktorzone (62) als Teil der zweiten Absorptionskolonne (55) und angeordnet im unteren Ende (63) der zweiten Absorptionskolonne (55) und/oder als Teil eines separaten Behälters,

wobei die Polysulfidreaktorzonen (42, 62) Pfropfenströmungszonen umfassen, wobei die Sulfidreaktorzonen (42, 62) ein stromaufwärts gelegenes Ende (44, 64) und ein stromabwärts gelegenes Ende (45, 65) umfassen, wobei das stromaufwärts gelegene Ende (44) der Polysulfidreaktorzone (42) der ersten Absorptionskolonne (35) mit dem Auslass (41) für die erste beladene wässrige Zwischenflüssigkeit fluidleitend verbunden ist und das stromaufwärts gelegene Ende (64) der Polysulfidreaktorzone (62) der zweiten Absorptionskolonne (55) mit dem Auslass (60) für die zweite beladene wässrige Zwischenflüssigkeit fluidleitend verbunden ist,

wobei das stromabwärtig gelegene Ende (45) der Polysulfidreaktorzone (42) der ersten Absorptionskolonne (35) mit dem stromaufwärtig gelegenen Ende (64) der Polysulfidreaktorzone (62) der zweiten Absorptionskolonne (55) fluidleitend verbunden ist,

wobei das stromabwärts gelegene Ende (65) der Polysulfidreaktorzone (62) der zweiten Absorptionskolonne (55) mit einem aeroben Bioreaktor (19) zur Oxidation von Sulfid zu elementarem Schwefel fluidleitend verbunden ist,

wobei der aerobe Bioreaktor (19) mit dem Einlass (59) für einen Teil (8a) der wässrigen alkalischen Flüssigkeit (8) der ersten Absorptionskolonne und mit dem Einlass (40) für einen Teil (8c) der wässrigen alkalischen Flüssigkeit (8) der zweiten fluidleitend verbunden ist, und

umfassend eine Einheit (22) zur Rückgewinnung von elementarem Schwefel, die mit einem Einlass versehen ist, der mit dem aeroben Bioreaktor (19) fluidleitend verbunden ist, und die mit einem Auslass (24) für elementaren Schwefel und einem Auslass (25) für einen an elementarem Schwefel armen flüssigen Abfluss versehen ist.

12. Anlage zur Rückgewinnung von Schwefel nach Anspruch 10 oder 11, wobei die Polysulfidreaktorzone (11, 42, 62) mit Mitteln zur Erhöhung der Temperatur des flüssigen Inhalts der Polysulfidreaktorzone (11, 42, 62) versehen ist.

13. Anlage zur Rückgewinnung von Schwefel nach einem der Ansprüche 10 bis 12 umfassend sulfidoxidierende Bakterien.

14. Verfahren nach Anspruch 6, durchgeführt in einer Anlage zur Rückgewinnung von Schwefel nach Anspruch 10.

15. Verfahren nach Anspruch 7, durchgeführt in einer Anlage zur Rückgewinnung von Schwefel nach Anspruch 11.

**Revendications**

1. - Procédé pour traiter en continu un gaz comprenant du sulfure d'hydrogène, ledit procédé comprenant les étapes suivantes :

a) mettre en contact le gaz comprenant du sulfure d'hydrogène avec un liquide alcalin aqueux comprenant des bactéries oxydant le sulfure et des particules de soufre élémentaire, produisant ainsi un liquide aqueux chargé comprenant du sulfure dissous, des composés polysulfures, des bactéries oxydant le sulfure et des particules de soufre élémentaire et un gaz ayant une teneur inférieure en sulfure d'hydrogène, et faire passer le liquide aqueux chargé à travers une zone de réacteur à polysulfure comprenant une ou plusieurs zones de réacteur à écoulement piston ;
b) mettre en contact le liquide aqueux chargé avec un oxydant pour permettre aux bactéries oxydant le sulfure d'oxyder le sulfure en soufre élémentaire, produisant ainsi un liquide aqueux enrichi comprenant une quantité accrue de particules de soufre élémentaire ; et
c) séparer les particules de soufre élémentaire du liquide aqueux enrichi,
dans lequel le temps de séjour du liquide aqueux chargé entre sa préparation à l'étape (a) et sa fourniture à l'étape (b) est entre 3 et 45 minutes ; et
dans lequel la teneur en soufre élémentaire en tant que partie des composés polysulfures dans le liquide aqueux chargé [$S^0$ dans $S_x^{2-}$] tel que fourni à l'étape (b) est au-dessus de 0,7 mM, de préférence au-dessus de 1 mM.

2. - Procédé selon la revendication 1, dans lequel, pendant une période d'au moins 1 semaine, la teneur moyenne quotidienne de soufre élémentaire en tant que partie des composés polysulfures dans le liquide aqueux chargé [$S^0$ dans $S_x^{2-}$] tel que fourni à l'étape (b) est au-dessus de 0,7 mM.

3. - Procédé selon l'une des revendications 1 ou 2, dans lequel la teneur en soufre élémentaire en tant que partie des composés polysulfures dans le liquide aqueux chargé satisfait la condition suivante :

$$[S^0 \text{ dans } S_x^{2-}] \geq 1{,}7 * [HS^-] * 10^{(-9{,}17+pH)},$$

dans laquelle [$S^0$ dans $S_x^{2-}$] est la teneur en soufre élémentaire en tant que partie des composés polysulfures dans le liquide aqueux chargé tel que fourni à l'étape (b) exprimée en mM, [HS⁻] est la concentration en sulfure exprimée en mM du liquide aqueux chargé tel que fourni à l'étape (b), et dans laquelle pH est le pH du liquide aqueux chargé tel que fourni à l'étape (b).

**4.** - Procédé selon l'une quelconque des revendications 1 à 3, dans lequel le gaz comprenant du sulfure d'hydrogène a une teneur en sulfure d'hydrogène d'entre 0,1 et 3 % en volume et une teneur en dioxyde de carbone d'au-dessus de 20 % en volume.

**5.** - Procédé selon l'une quelconque des revendications 1 à 4, dans lequel l'étape (a) comprend l'opération consistant à faire passer le liquide aqueux chargé à travers une zone de réacteur à polysulfure comprenant une ou plusieurs zones de réacteur à écoulement piston, la zone de réacteur de polysulfure ayant une région amont et une région aval, et dans lequel, de préférence, dans la zone de réacteur à polysulfure, une partie du liquide aqueux chargé est recyclée de la région aval vers la région amont dans la zone de réacteur de polysulfure.

**6.** - Procédé selon l'une quelconque des revendications 1 à 5, dans lequel l'étape (a) est réalisée dans une colonne verticale, le gaz comprenant du sulfure d'hydrogène étant introduit en continu dans la colonne à une position inférieure de la colonne et le liquide aqueux comprenant des bactéries oxydant le sulfure est introduit en continu à une position supérieure de la colonne de telle sorte qu'un courant gazeux s'écoulant sensiblement vers le haut entre en contact avec un courant aqueux s'écoulant sensiblement vers le bas.

**7.** - Procédé selon l'une quelconque des revendications 1 à 3, dans lequel, en tant que partie de l'étape (a), une partie du liquide aqueux comprenant des bactéries oxydant le sulfure est mise en contact en continu dans une étape (a1) avec le gaz comprenant du sulfure d'hydrogène pour obtenir un premier liquide aqueux chargé intermédiaire et un gaz intermédiaire ayant une teneur intermédiaire inférieure en sulfure d'hydrogène,

dans lequel, en tant que partie de l'étape (a), une autre partie du liquide aqueux comprenant des bactéries oxydant le sulfure est mise en contact en continu dans une étape (a2) avec le gaz intermédiaire ayant une teneur intermédiaire inférieure en sulfure d'hydrogène pour obtenir un second liquide aqueux chargé intermédiaire et le gaz ayant une teneur inférieure en sulfure d'hydrogène, et
dans lequel le premier liquide aqueux chargé intermédiaire est combiné avec le second liquide aqueux chargé intermédiaire pour obtenir le liquide aqueux chargé.

**8.** - Procédé selon la revendication 7, dans lequel les premier et second liquides aqueux chargés intermédiaires s'écoulent respectivement à travers des première et seconde zones de réacteur à polysulfure séparées, dans lesquelles zones de réacteur à polysulfure des composés polysulfures sont formés par réaction du sulfure dissous et du soufre élémentaire.

**9.** - Procédé selon l'une quelconque des revendications 1 à 8, dans lequel au moins une partie du liquide aqueux enrichi de l'étape (b) est recyclée à l'étape (a).

**10.** - Installation de traitement de récupération de soufre (1) comprenant :

- une colonne d'absorption (2) comportant une entrée (3) pour un gaz comprenant du sulfure d'hydrogène (4), une sortie (5) pour un gaz (6) ayant une teneur inférieure en sulfure d'hydrogène à son extrémité supérieure, une entrée (7) pour un liquide alcalin aqueux (8) comprenant en outre des bactéries oxydant le sulfure et une première sortie (9) pour un liquide aqueux chargé (10) à une hauteur inférieure ;
- une zone de réacteur à polysulfure (11) en tant que partie de la colonne d'absorption (2) et positionnée dans l'extrémité inférieure (2a) de la colonne d'absorption (2) et/ou en tant que partie d'un récipient séparé, la zone de réacteur à polysulfure (11) comprenant une ou plusieurs zones de réacteur à écoulement piston, ladite zone de réacteur à polysulfure (11) comprenant une extrémité amont (13) et une extrémité aval (14) ;
l'extrémité amont (13) de la zone de réacteur à polysulfure (11) étant reliée fluidiquement à la première sortie (9) pour un liquide aqueux chargé (10) ;
l'extrémité aval (14) de la zone de réacteur à polysulfure (11) comportant une seconde sortie (16) pour le liquide aqueux chargé (17) et un courant de recyclage (18) pour une partie du liquide aqueux chargé à l'extrémité amont (13) de la zone de réacteur à polysulfure (11), ledit courant de recyclage comprenant, de préférence, un récipient pour augmenter le temps de séjour dans le courant de recyclage ;
la seconde sortie (16) pour le liquide aqueux chargé (17) étant reliée fluidiquement à un bioréacteur aérobie (19) pour l'oxydation du sulfure en soufre élémentaire ;

le bioréacteur aérobie (19) étant relié fluidiquement à l'entrée (7) pour un liquide alcalin aqueux (8) de la colonne d'absorption (2) ;
et comprenant une unité (22) de récupération de soufre élémentaire comportant une entrée reliée fluidiquement au bioréacteur aérobie (19) et comportant une sortie (24) pour le soufre élémentaire et une sortie (25) pour un effluent liquide pauvre en soufre élémentaire.

11. - Installation de récupération de soufre (1a) comprenant :

- une première colonne d'absorption (35) comportant une entrée (36) pour un gaz comprenant du sulfure d'hydrogène (4), une sortie (37) pour un gaz intermédiaire (38) ayant une teneur inférieure en sulfure d'hydrogène à son extrémité supérieure (39), une entrée (40) pour une partie (8c) d'un liquide alcalin aqueux (8) comprenant en outre des bactéries oxydant le sulfure et une sortie (41) pour un premier liquide aqueux chargé intermédiaire à une hauteur inférieure ;
- une seconde colonne d'absorption (55) comportant une entrée (56) pour le gaz intermédiaire (38) ayant une teneur inférieure en sulfure d'hydrogène, une sortie (57) pour un gaz (6) ayant une teneur inférieure en sulfure d'hydrogène à son extrémité supérieure (58), une entrée (59) pour une partie (8a) d'un liquide alcalin aqueux (8) comprenant en outre des bactéries oxydant le sulfure et une sortie (60) pour un second liquide aqueux chargé intermédiaire à une hauteur inférieure ;
- une zone de réacteur à polysulfure (42) en tant que partie de la première colonne d'absorption (35) et positionnée dans l'extrémité inférieure (43) de la première colonne d'absorption (35) et/ou en tant que partie d'un récipient séparé ;
- une zone de réacteur à polysulfure (62) en tant que partie de la seconde colonne d'absorption (55) et positionnée dans l'extrémité inférieure (63) de la seconde colonne d'absorption (55) et/ou en tant que partie d'un récipient séparé ;
les zones de réacteur à polysulfure (42, 62) comprenant des zones à écoulement piston, lesdites zones de réacteur à sulfure (42, 62) comprenant une extrémité amont (44, 64) et une extrémité aval (45, 65), l'extrémité amont (44) de la zone de réacteur à polysulfure (42) de la première colonne d'absorption (35) étant reliée fluidiquement à la sortie (41) pour le premier liquide aqueux chargé intermédiaire et l'extrémité amont (64) de la zone de réacteur à polysulfure (62) de la seconde colonne d'absorption (55) étant reliée fluidiquement à la sortie (60) pour le second liquide aqueux chargé intermédiaire,
l'extrémité aval (45) de la zone de réacteur à polysulfure (42) de la première colonne d'absorption (35) étant reliée fluidiquement à l'extrémité amont (64) de la zone de réacteur à polysulfure (62) de la seconde colonne d'absorption (55) ; l'extrémité aval (65) de la zone de réacteur à polysulfure (62) de la seconde colonne d'absorption (55) étant reliée fluidiquement à un bioréacteur aérobie (19) pour l'oxydation de sulfure en soufre élémentaire ;
le bioréacteur aérobie (19) étant relié fluidiquement à l'entrée (59) pour une partie (8a) du liquide alcalin aqueux (8) de la première colonne d'absorption et relié fluidiquement à l'entrée (40) pour une partie (8c) du liquide alcalin aqueux (8) de la seconde colonne d'absorption ; et
comprenant une unité de récupération de soufre élémentaire (22) comportant une entrée reliée fluidiquement au bioréacteur aérobie (19) et comportant une sortie (24) pour le soufre élémentaire et une sortie (25) pour un effluent liquide pauvre en soufre élémentaire.

12. - Installation de traitement de récupération de soufre selon l'une des revendications 10 ou 11, dans laquelle la zone de réacteur à polysulfure (11, 42, 62) comporte des moyens pour augmenter la température du contenu liquide de la zone de réacteur à polysulfure (11, 42, 62).

13. - Installation de traitement de récupération de soufre selon l'une quelconque des revendications 10 à 12, comprenant des bactéries oxydant le sulfure.

14. - Procédé selon la revendication 6 tel que mis en œuvre dans une installation de traitement de récupération de soufre selon la revendication 10.

15. - Procédé selon la revendication 7 tel que mis en œuvre dans une installation de traitement de récupération de soufre selon la revendication 11.

Fig. 1

Fig. 2

Comp.A      Ex. 1

Ex. 2      Ex. 3

Fig. 3

Fig. 4

Fig. 5

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9210270 A

- WO 9210270 H

**Non-patent literature cited in the description**

- **KLEINJAN, W. E** ; **DE KEIZER, A** ; **JANSSEN, A. J. H**. Equilibrium of the reaction between dissolved sodium sulphide and biologically produced sulphur. *Colloids and Surfaces B: Biointerfaces*, 2005, vol. 43 (3-4), 228-237

- **ALEXEY KAMYSHNY** ; **JENNY GUN** ; **DAN RIZKOV** ; **TAMARA VOITSEKOVSKI** ; **OVADIA LEV**. *Environ. Sci. Technol*, 2007, vol. 41 (7), 2395-2400
- **PFENNIG, N** ; **LIPPERT, K.D**. Uber das Vitamin B12-bedurfnis phototropher Schwefelbacterien. *Arch. Microbiol*, 1966, vol. 55, 245-256